# EUROPEAN PATENT SPECIFICATION

(11) **EP 2 344 085 B1**
(45) Date of publication and mention of the grant of the patent: **16.11.2016**
(21) Application number: 09785011.9
(22) Date of filing: 27.08.2009
(51) Int. Cl.: A61F 2/34, A61F 2/30

(54) **A PROSTHETIC BEARING COMPONENT**
PROTHESENLAGERKOMPONENTE
PIÈCE D'APPUI DE PROTHÈSE

(30) Priority: 01.09.2008 GB 0815884
(43) Date of publication of application: 20.07.2011
(73) Proprietor: Biomet UK Limited, Bridgend South Wales CF31 3XA (GB)
(72) Inventor: KHAN, Mohammed, Imran, Berkshire RG1 3PP (GB); BIGSBY, Robert, John, Andrew, Penarth CF64 1EJ (GB); SCOTT, Robert, Andrew, Chippenham Wiltshire SN15 4BX (GB); SCHROEDER, David, Wayne, Winona Lake IN 46590 (US)
(74) Representative: Mays, Julie
(86) International application number: PCT/GB2009/002077
(87) International publication number: WO 2010/023447

(56) References cited:
- EP-A- 1 338 256
- EP-A- 1 647 242
- EP-A2- 2 249 887
- WO-A-95/15734
- FR-A- 2 838 329
- US-A- 5 370 702
- US-A1- 2007 038 304

## Description

This invention relates to a prosthetic bearing component and particularly but not exclusively relates to a prosthetic bearing component with one or more openings, which are adapted to receive attachment means for attaching the prosthesis to bone.

### Background

When replacing part or all of a human or animal joint with a prosthesis, it is often desirable to screw or pin a bearing component directly to a bone as an alternative to, or in addition to, using cement, particularly in cases where the bone has become degraded. For example, in hip replacement surgery, the acetabulum may be in a poor condition and a cemented joint between a prosthetic acetabular cup and the acetabulum may not be sufficient. The prosthetic acetabular cup may therefore require direct fixation to the acetabulum using screws, pins or other such securing means.

Where direct fixation through the bearing component is desirable, a liner between the bearing components is necessary. For example, conventional metal-on-metal or ceramic-on-ceramic bearings rely on mixed-mode and fluid film lubrication between components. Such lubrication modes require smooth bearing surfaces and cannot tolerate fixation components or openings on a bearing surface. A liner is therefore required to cover the fixation components or openings in order to ensure effective lubrication between components. However, the presence of this liner reduces the contact area between the bearing components, which increases the stresses and hence wear rates, and also reduces the stability of the prosthesis.

Furthermore, metal-on-metal bearings are often desirable as they have a proven track record and they allow large diameter bearings which reduce wear rates and improve stability. However, some concerns have been expressed in the literature over possible detrimental effects of metal ions released into the body from such bearings.

In addition to the above, many designs of acetabular shells with separate liners incorporate holes to allow the placement of screws to enhance primary fixation to the underlying bone. However, the pumping action of the joint fluid may contribute to the pathogenesis of osteolysis. For example, in Walter et al. 2004, ten patients who were scheduled for revision for pelvic osteolysis were studied. All had bone-ingrown metal-backed cups with holes and polyethylene liners. Pressures were measured in the osteolytic lesion and in the hip joint while applying cyclic forces across the artificial joint. In four cases with lesions that were fully contained by bone, loading of the hip produced a pressure wave in the osteolytic lesion. Cyclic forces, such as those that occur in normal gait, can act on the polyethylene liner, the metal shell, and the supporting bone to pump fluid in the retroacetabular osteolytic lesion. This pumping action may contribute to the pathogenesis of osteolysis by the mechanisms of fluid pressure, fluid flow, or the transportation of wear particles.

Similarly, in Walter et al. 2005 it was found that the pumping of fluid and polyethylene wear debris from the joint space to the retroacetabular bone is implicated in the pathogenesis of osteolysis. Three possible mechanisms for this pumping: pressure gradients, diaphragm pumping, and piston pumping were studied in vitro in a laboratory model. The simulated activities of rising from a chair and climbing stairs produced high-pressure gradients and high angles of loading that could pump fluid through the apical hole to the retroacetabular bone. A noncongruent liner acted as a diaphragm pump, producing pressures six times higher than that seen with a congruent liner. Pistoning motion of the liner produced pressures eight times higher than when no pistoning occurs. These pumping mechanisms could be mitigated by the use of acetabular components without holes.

FR2838329A discloses an acetabular cup comprising openings for receiving screws to fix the acetabular cup in place. However, the acetabular cup disclosed in FR2838329A requires a separate liner for receiving the femoral head.

EP1647242A1 discloses the features of the preamble of claim 1. It shows a prosthetic acetabular cup with a bearing surface layer made from a composite material comprises PEEK resin and at least 20 to 40% short carbon fibres.

The present invention therefore seeks to address these issues.

### References

Walter et al. 2004: J Arthroplasty. 2004 Feb;19(2):230-4. "The pumping of fluid in cementless cups with holes". Walter WL, Walter WK, O'Sullivan M.

Walter et al. 2005: J Arthroplasty. 2005 Dec;20(8):1042-8. "Mechanisms for pumping fluid through cementless acetabular components with holes". Walter WL, Clabeaux J, Wright TM, Walsh W, Walter WK, Sculco TP.

### Statements of Invention

According to a first aspect of the present invention, there is provided a prosthetic bearing component comprising a bearing surface made from Carbon-Fibre-Reinforced Polyaryletheretherketone (CFR-PEEK), wherein the prosthetic bearing component has one or more openings comprising a frangible element, the frangible element being selectively breakable such that one or more of the openings are provided on the bearing surface and wherein the one or more of the openings are suitable for receiving one or more attachment means to secure the prosthetic bearing component in place.

The one or more openings may be reinforced in use by one or more of a cap, the attachment means and a frangible element.

One or more of the openings may continue through the thickness of the prosthetic bearing component. Alternatively and/or additionally, one or more of the openings may extend partially through the thickness of the prosthetic bearing component.

One or more of the openings may comprise a blind bore or an annular recess. An engagement portion for engagement with an engagement means may be provided inside the blind bore or annular recess. One or more of the openings may comprise an annular groove. An engagement portion for engagement with an engagement means may be provided within the annulus defined by the annular groove. The engagement portion may be defined by a recess. The engagement portion may be defined by a projection.

One or more of the openings comprise a frangible element. One or more of the openings may start from an outer surface of the prosthetic bearing component and the one or more openings may continue through the thickness of the prosthetic bearing component when the frangible element is selectively broken. One or more of the openings may start from an inner surface of the prosthetic bearing component and the one or more openings may continue through the thickness of the prosthetic bearing component when the frangible element is selectively broken.

The perimeter of the one or more openings on the bearing surface may be continuous such that the openings are enclosed by the bearing surface. One or more caps may be provided in the one or more openings in the bearing surface.

The attachment means may comprise a head and the head of the attachment means may not be flush with the bearing surface such that a recess may be formed in the bearing surface. The one or more attachment means may be screws. Alternatively, the one or more attachment means may be pins.

The one or more attachment means may be made from a material that, when in contact with another component, may operate under boundary lubrication. The bearing surface may be made from a material that, when in contact with another component, may operate under boundary lubrication. The one or more caps may be made from a material that, when in contact with another component, may operate under boundary lubrication. The material which may operate under boundary lubrication may be CFR-PEEK.

The prosthetic bearing component may comprise a single layer of CFR-PEEK. In addition, one or more of a porous coating, a hydroxyapatite (HA) coating and a porous titanium bone in-growth surface comprising interconnected porosity may be provided on the surface of the prosthetic bearing component opposite the bearing surface.

A kit of parts for a prosthetic bearing may comprise (a) the above-mentioned prosthetic bearing component; and (b) one or more attachment means, wherein the one or more attachment means pass through the one or more openings to secure the prosthetic bearing component to bone.

One or more of the openings may continue through the prosthetic bearing component. Alternatively and/or additionally, one or more of the openings may extend partially through the thickness of the prosthetic bearing component.

The kit of parts may further comprise one or more caps, wherein the one or more caps may be suitable for being provided in the one or more openings in the bearing surface. The one or more caps may be made from CFR-PEEK. The bearing surface and/or the one or more attachment means may also be made from CFR-PEEK.

The prosthetic bearing component may be a prosthetic acetabular cup.

### Brief Description of the Drawings

For a better understanding of the present invention, and to show more clearly how it may be carried into effect, reference will now be made, by way of example, to the following drawings, in which:
Figure 1 is a sectional side view of a prosthetic acetabular cup according to a first embodiment of the present invention;
Figure 2 is a sectional side view of a prosthetic acetabular cup showing a schematic of a partial opening according to a second or a third embodiment of the present invention;
Figures 3a-k are sectional side views of the encircled portion of Figure 2 showing various profile shapes according to the second embodiment of the invention;
Figures 4a-o are sectional side view of the encircled portion of Figure 2 showing various profile shapes according to the third embodiment of the invention; and
Figures 5a-i are sectional side views of the encircled portion of Figure 2 showing various removal methods for the second and third embodiments of the invention.

### Detailed Description

The present invention relates to a cementless monoblock acetabular cup with openings in the bearing surface and attachment means which pass through the openings to secure the acetabular cup to the acetabulum.

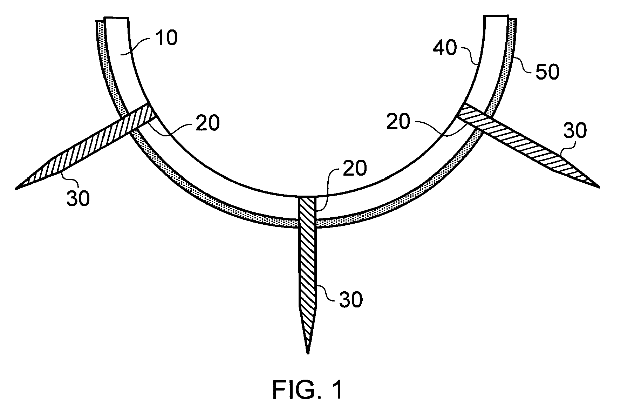

With reference to Figure 1, a prosthetic bearing component according to a first embodiment of the invention comprises a substantially hemispherical acetabular cup 10 with one or more openings 20. The acetabular cup 10 is a large diameter monoblock cup. As such, an inner surface 40 of the acetabular cup 10 provides a bearing surface, which interfaces directly with a substantially spherical head of femoral bearing component (not shown). The acetabular cup 10 is manufactured from Carbon-Fibre-Reinforced Polyaryletheretherketone (CFR-PEEK), which provides a low wear bearing surface. The acetabular cup 10 is machined from bar or preferably injection moulded. An outer surface 50 of the acetabular cup 10 is porous coated and/or hydroxyapatite (HA) coated or is provided with a porous titanium bone in-growth surface comprising interconnected porosity. The porous coating may be achieved by using thermal spraying (for example, using plasma spraying, arc spraying, flame spraying or using a high velocity oxyfuel) or cold gas spraying. The hydroxyapatite (HA) coat may be achieved by using thermal spraying, cold gas spraying or electrochemically assisted deposition.

One or more attachment means 30 are also provided. The one or more attachment means 30 attach the acetabular cup 10 to an acetabulum (not shown) by passing through the one or more openings 20. The attachment means 30 may be screws, pins, barbs, spikes or any other suitable attachment device. Furthermore, to provide enhanced fixation, the acetabular cup 10 may have additional attachment means provided on the outer surface 50 of the acetabular cup 10, which do not pass through the one or more openings 20.

The attachment means 30 are also manufactured from CFR-PEEK (for example with a continuous fibre orientation). The bearing surface of the femoral head should not therefore be damaged by the attachment means 30 as they are manufactured from the same material as the acetabular cup 10.

One or more caps (not shown) may also be provided. The caps are adapted to block off the openings 20 if the surgeon does not wish to use attachment means 30 through a particular opening 20. The caps may also be manufactured from CFR-PEEK.

CFR-PEEK operates under boundary lubrication and so the presence of openings does not affect the wear performance of the bearing. By placing openings in the bearing surface it is possible to achieve high wear performance and also enhanced fixation if bone quality is poor. The present invention therefore provides a single low wear solution that is suitable for all types of patients and bone quality. Furthermore, because there is no exposed metal involved in the articulation there is no production of metal ions.

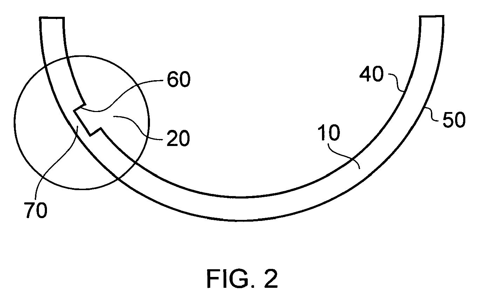

With reference to Figure 2, a prosthetic bearing component according to a second embodiment of the invention comprises one or more openings which extend partially through the thickness of the acetabular cup, such that one or more of the openings comprises a blind bore 60. This contrasts with the first embodiment described above, in which the openings 20 continue through the thickness of the acetabular cup 10. The blind bore 60 may extend into either the outer surface 50 or the inner surface 40 of the acetabular cup 10. Furthermore, in the second embodiment one or more of the openings comprises a frangible element 70 such that when the frangible element 70 is broken the opening will continue through the full thickness of the acetabular cup 10. (For example, the frangible element may comprise the bottom of a blind bore.)

The surgeon can therefore select which of the frangible openings to break out. For example, the Surgeon may choose which elements to remove during the surgery should further fixation be required, prior to insertion of the component into the acetabulum or even in situ after press-fitting the component. The remaining openings remain closed, thereby minimising the number of openings used to the requirements of the patient. It is advantageous, although not essential, for the blind bore to extend into the inner surface 40 of the acetabular cup 10, so that any peripheral damage caused by breaking out the frangible element will be caused to the outer surface 60, not the inner bearing surface 40.

The openings 20 may be reinforced during use by one or more of the cap, the attachment means 30 and the frangible element 70. Such reinforcements could compensate for the notch sensitivity of CFR-PEEK.

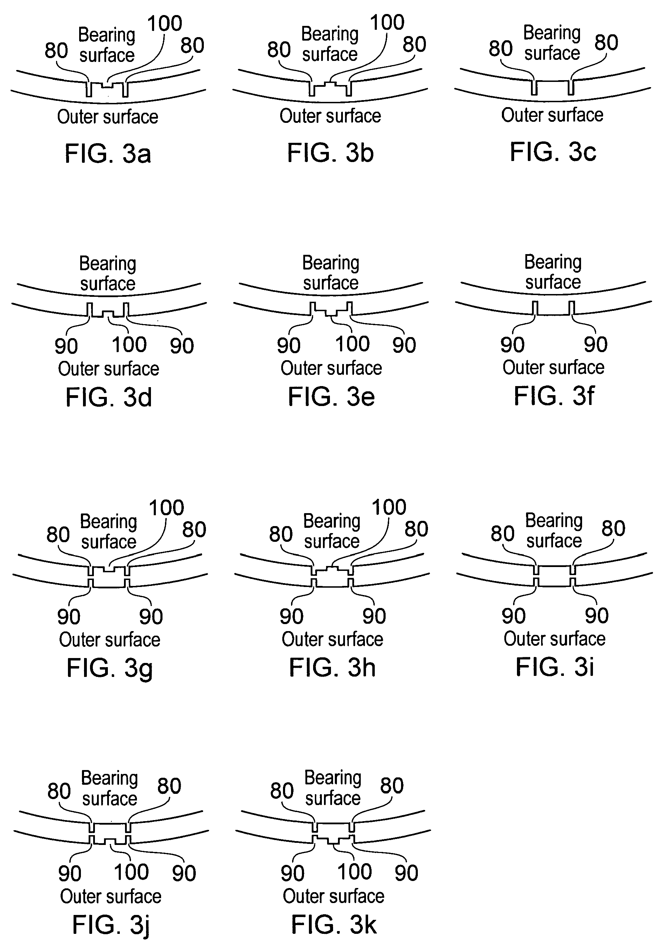

With reference to Figure 3, a prosthetic bearing component according to a second embodiment of the invention comprises an annular groove 80 on the inner surface 40 of the acetabular cup 10 (as shown in Figures 3a-c) or an annular groove 90 on the outer surface 50 of the acetabular cup 10 (as shown in Figures 3d-f). Alternatively, the annular grooves 80, 90 may be provided on both the inner surface 40 and the outer surface 50 of the acetabular cup 10 (as shown in Figures 3g-k).

The annular grooves 80, 90 define a perimeter on the inner and/or outer surfaces 40, 50. Figure 3 shows a section through the thickness of the acetabular cup 10 and the annular grooves 80, 90 such that two opposite ends of the same annular grooves 80, 90 are shown. The perimeter of the annular grooves 80, 90 may be circular, square, star-shaped, cross-shaped or any other shape. The perimeters of the annular groves 80, 90 are shaped so as to accommodate the one or more attachment means 30.

The annular grooves 80, 90 reduce the thickness of the acetabular cup 10 locally and therefore define a line of weakness in the acetabular cup so as to provide the frangible element 70. The portion of the acetabular cup 10 inside the perimeter defined by the annular grooves 80, 90 can therefore be removed by breaking the frangible element 70.

The prosthetic bearing component according to the second embodiment of the invention may also comprise an engaging portion 100. The engaging portion 100 may either be in the form of a recess (as shown in Figures 3a, 3d, 3g and 3j) or in the form of a protrusion (as shown in Figures 3b, 3e, 3h and 3k). Alternatively, there may be no engagement portion (as shown in Figures 3c, 3f and 3i). The engaging portion 100 is provided within the perimeter of the annular groove 80, 90, and may be provided on the inner surface 40 or outer surface 50 of the acetabular cup 10.

To assist removal of the frangible element 70, the engaging portion 100 is shaped to receive an engaging means to allow instumented removal. For example, the engaging portion may be shaped to include a variety of screw drive types such as: slotted, Phillips ("Crosshead"), Pozidriv (SupaDriv), Torx, Hex (Allen), Robertson, Tri-Wing, Torq-Set, Spanner Head, Triple Square (XZN), Polydrive, One-way, etc.

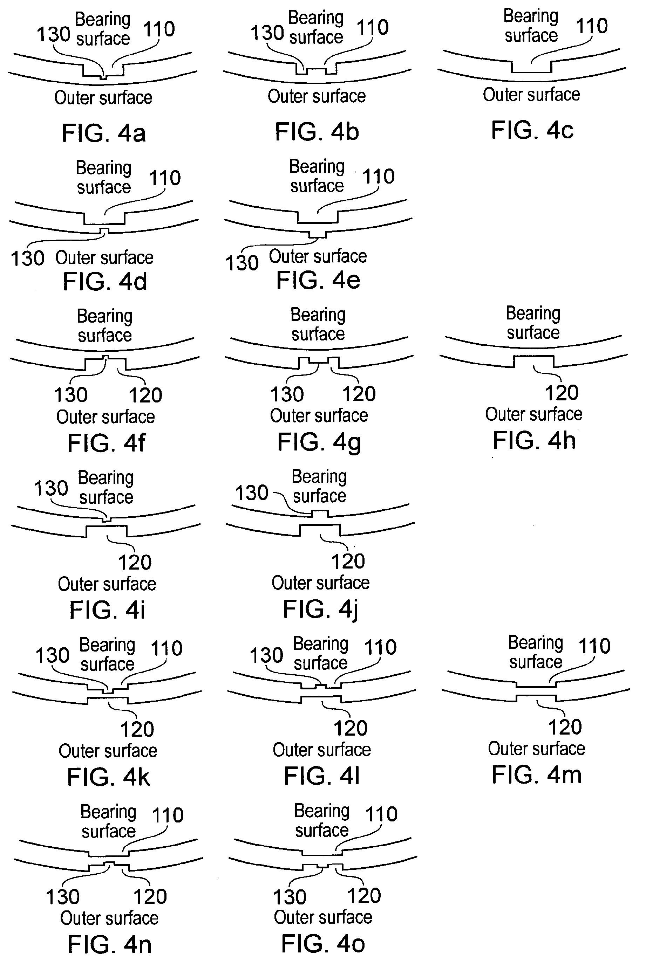

With reference to Figure 4, a prosthetic bearing component according to a third embodiment of the invention comprises an annular recess portion 110 on the inner surface 40 of the acetabular cup 10 (as shown in Figures 4a-e) or an annular recess portion 120 on the outer surface 50 of the acetabular cup 10 (as shown in Figures 4f-j). Alternatively, the annular recess portions 110, 120 may be provided on both the inner surface 40 and the outer surface 50 of the acetabular cup 10 (as shown in Figures 4k-o).

The annular recess portions 110, 120 define a perimeter on the inner and/or outer surfaces 40, 50. Figure 4 shows a section through the thickness of the acetabular cup 10 and the annular recess portions 110, 120. The perimeter of the annular grooves 80, 90 may be circular, square, star-shaped, cross-shaped or any other shape. The perimeters of the annular recess portions 110, 120 are shaped so as to accommodate the one or more attachment means 30.

The annular recess portions 110, 120 locally reduce the thickness of the acetabular cup 10 and therefore define a line of weakness in the acetabular cup so as to provide the frangible element 70. The portion of the acetabular cup 10 inside the perimeter defined by the annular recess portions 110, 120 can therefore be removed by breaking the frangible element 70.

The prosthetic bearing component according to the third embodiment of the invention may also comprise an engaging portion 130. The engaging portion 130 may either be in the form of a recess (as shown in Figures 4a, 4d, 4f, 4i, 4k and 4n) or in the form of a protrusion (as shown in Figures 4b, 4e, 4g, 4j, 4l and 4o). Alternatively, there may be no engagement portion (as shown in Figures 4c, 4h and 4m). The engaging portion 130 is provided within the perimeter of one, or both, of the annular recess portions 110, 120 and the engaging portion 130 may be provided on the inner surface 40 or outer surface 50 of the acetabular cup 10. The engaging portion 130, whether a recess of a protrusion, may be provided on the opposite surface of the acetabular cup 10 to the annular recess portion 100, 120 (as shown in Figures 4d, 4e, 4i, 4j).

As for the second embodiment, to assist removal of the frangible element 70, the engaging portion 130 is shaped to receive an engaging means to allow instumented removal. The engaging portion 130 of the third embodiment may be shaped to include the same variety of screw drive types as for the second embodiment.

The inside walls of the annular recess portions 110, 120 and/or annular grooves 80, 90 of the second and third embodiments may also have screw threads to allow locking of screws at specific angles to provide angle stable fixation.

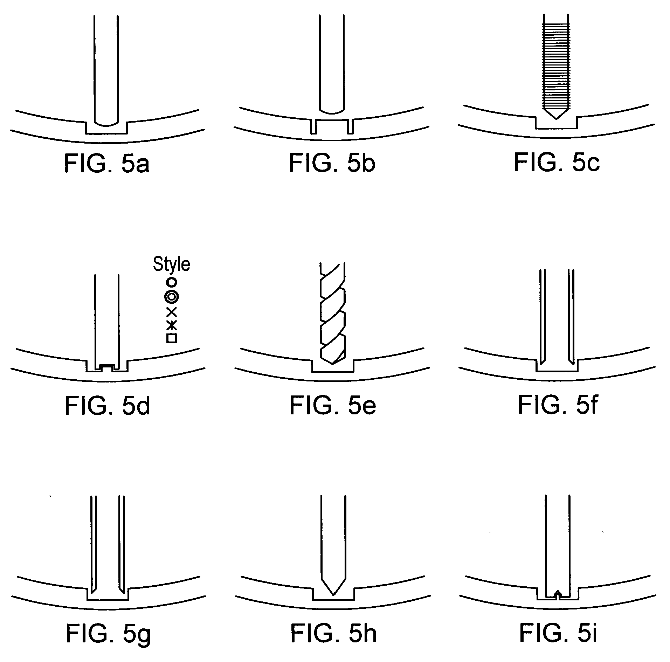

With reference to Figure 5, various methods for removing the frangible element are shown. These elements may be removed through a variety of means. For example, methods of removal include pushing out (Figures 5a and 5b), screwing through (Figure 5c), twisting out using the engaging portions 100,130 and the engagement means described above (Figure 5d), drilling out (Figure 5e), coring out (Figure 5f), punching out with a hollow tubular (Figure 5g), punching out with a pointed shaft (Figure 5h) and pulling out (Figure 5i). This list is not exhaustive and other removal techniques may be used. Furthermore, any of the above techniques could be applied to any of the frangible elements of the second and third embodiments described above (i.e. with an annular groove or annular recess portion).

The above-mentioned embodiments involve machining 'blank' elements (frangible elements) in the inner bearing or outer wall surface of the acetabular cup, which may be removed by the Surgeon during surgery to allow the placement of screws or spikes to enhance fixation. If the frangible elements are not removed, they continue to seal the bearing surface, preventing the transmission of fluid or wear particles into the acetabulum and also provide additional strength to the acetabular cup. The removable frangible elements may also be formed by machining the blind holes or annular grooves through part of the wall thickness of the acetabular cup.

The present invention may be used for modular cementless shells, but the preferred application is a CFR-PEEK monoblock cup, which can provide low wear when articulating against a metal or ceramic head even with the presence of screw holes in the bearing surface.

## Claims

1. A prosthetic bearing component (10) comprising a bearing surface (40) made from Carbon-Fibre-Reinforced Polyaryletheretherketone (CFR-PEEK), **characterised in that** the prosthetic bearing component has one or more openings (20) comprising a frangible element (70), the frangible element being selectively breakable such that one or more of the openings (20) are provided on the bearing surface and wherein the one or more of the openings are suitable for receiving one or more attachment means (30) to secure the prosthetic bearing component in place.

2. The prosthetic bearing component as claimed in claim 1, wherein the one or
more openings (20) are reinforced in use by one or more of a cap, the attachment means (30) and a frangible element (20).

3. The prosthetic bearing component as claimed in claim 1 or 2, wherein one or
more of the openings (20) continue through the thickness of the prosthetic bearing component (10).

4. The prosthetic bearing component as claimed in any preceding claim, wherein
one or more of the openings (20) extend partially through the thickness of the prosthetic bearing component.

5. The prosthetic bearing component as claimed in claim 4, wherein one or more of the openings (20) is formed by an annular recess (110, 120).

6. The prosthetic bearing component as claimed in claim 5, wherein an engagement portion (100, 130) for engagement with an engagement means is provided inside the annular recess (110, 120).

7. The prosthetic bearing component as claimed in claim 4, wherein one or more of the openings (20) is formed by an annular groove (80, 90).

8. The prosthetic bearing component as claimed in claim 7, wherein an engagement portion (100) for engagement with an engagement means is provided within the annulus defined by the annular groove (80, 90).

9. The prosthetic bearing component as claimed in claim 6 or 8, wherein the engagement portion (100, 130) is defined by a recess or by a projection.

10. The prosthetic bearing component as claimed in claim 1, wherein one or more of the openings (20) starts from an outer surface (50) or an inner surface (40) of the prosthetic bearing component and the one or more openings continue through the thickness of the prosthetic bearing component when the frangible element (70) is selectively broken.

11. The prosthetic bearing component as claimed in any preceding claim, wherein the perimeter of the one or more openings (20) on the bearing surface is continuous such that the openings are enclosed by the bearing surface.

12. The prosthetic bearing component as claimed in any preceding claim, wherein the attachment means (30) comprises a head and the head of the attachment means is not flush with the bearing surface such that a recess is formed in the bearing surface.

13. The prosthetic bearing component as claimed in any preceding claim, wherein one or more caps are provided in the one or more openings (20) in the bearing surface.

14. The prosthetic bearing component as claimed in any preceding claim, wherein the prosthetic bearing component comprises a single layer of CFR-PEEK.

## Patentansprüche

1. Gelenkprothesenkomponente (10), umfassend eine Auflagerfläche (40) bestehend aus kohlenstofffaserverstärktem Polyaryletheretherketon (CFR-PEEK), **dadurch gekennzeichnet, dass** die Gelenkprothesenkomponente eine oder mehrere Öffnungen (20) aufweist, umfassend ein zerbrechliches Element (70), wobei das zerbrechliche Element selektiv zerbrechlich ist, so dass eine oder mehrere der Öffnungen (20) an der Auflagerfläche vorgesehen werden, und wobei sich die eine oder die mehreren Öffnungen eignen, ein oder mehrere Befestigungsmittel (30) aufzunehmen, um die Gelenkprothesenkomponente zu befestigen.

2. Gelenkprothesenkomponente nach Anspruch 1, wobei die eine oder die mehreren Öffnungen (20) während der Verwendung durch eines oder mehrere der Folgenden verstärkt sind: ein Deckel, ein Befestigungsmittel (30) und ein zerbrechliches Element (70).

3. Gelenkprothesenkomponente nach Anspruch 1 oder 2, wobei eine oder mehrere der Öffnungen (20) durch die Stärke der Gelenkprothesenkomponente (10) hindurch gehen.

4. Gelenkprothesenkomponente nach einem der vorstehenden Ansprüche, wobei sich eine oder mehrere der Öffnungen (20) teilweise durch die Stärke der Gelenkprothesenkomponente erstrecken.

5. Gelenkprothesenkomponente nach Anspruch 4, wobei eine oder mehrere der Öffnungen (20) durch eine kreisrunde Aussparung (110, 120) gebildet wird/werden.

6. Gelenkprothesenkomponente nach Anspruch 5, wobei ein Einrastanteil (100, 130) zum Einrasten mit einem Einrastmittel in der kreisrunden Aussparung (110, 120) vorgesehen ist.

7. Gelenkprothesenkomponente nach Anspruch 4, wobei eine oder mehrere der Öffnungen (20) durch eine kreisrunde Kerbe (80, 90) gebildet werden.

8. Gelenkprothesenkomponente nach Anspruch 7, wobei ein Einrastanteil (100) zum Einrasten mit einem Einrastmittel im Kreis vorgesehen ist, der durch die kreisrunde Kerbe (80, 90) definiert wird.

9. Gelenkprothesenkomponente nach Anspruch 6 oder 8, wobei der Einrastanteil (100, 130) durch eine Aussparung oder einen Ansatz definiert ist.

10. Gelenkprothesenkomponente nach Anspruch 1, wobei eine oder mehrere der Öffnungen (20) an einer Außenfläche (50) oder einer Innenfläche (40) der Gelenkprothesenkomponente beginnt/beginnen und die eine oder die mehreren Öffnungen durch die Stärke der Gelenkprothesenkomponente hindurch geht/gehen, wenn das zerbrechliche Element (70) selektiv zerbrochen wird.

11. Gelenkprothesenkomponente nach einem der vorstehenden Ansprüche, wobei der Umfang der einen oder der mehreren Öffnungen (20) entlang der Auflagerfläche durchgehend ist, so dass die Öffnungen durch die Auflagerfläche eingeschlossen sind.

12. Gelenkprothesenkomponente nach einem der vorstehenden Ansprüche, wobei das Befestigungsmittel (30) einen Kopf umfasst und der Kopf des Befestigungsmittels nicht mit der Auflagerfläche bündig ist, so dass in der Auflagerfläche eine Aussparung entsteht.

13. Gelenkprothesenkomponente nach einem der vorstehenden Ansprüche, wobei ein oder mehrere Deckel in der einen oder den mehreren Öffnungen (20) in der Auflagerfläche vorgesehen sind.

14. Gelenkprothesenkomponente nach einem der vorstehenden Ansprüche, wobei die Gelenkprothesenkomponente eine einzelne Schicht aus CFR-PEEK umfasst.

## Revendications

1. Pièce d'appui de prothèse (10) comprenant une surface d'appui (40) réalisée en polyaryléthercétone (PAEK) renforcé à la fibre de carbone, **caractérisé en ce que** la pièce d'appui de prothèse présente une ou plusieurs ouvertures (20) comprenant un élément frangible (70), l'élément frangible étant sélectivement cassable de sorte qu'une ou plusieurs des ouvertures (20) soient pratiquées sur la surface d'appui, et la ou plusieurs des ouvertures soit appropriée pour recevoir un ou plusieurs dispositifs de fixation (30) pour la fixation d'une pièce d'appui de prothèse en place.

2. Pièce d'appui de prothèse selon la revendication 1, la ou plusieurs ouvertures (20) étant renforcées en cours d'usage par un ou plusieurs des suivants : un bouchon, le dispositif de fixation (30) et un élément frangible (70).

3. Pièce d'appui de prothèse selon la revendication 1 ou 2, une ou plusieurs des ouvertures (20) se prolongeant à travers l'épaisseur de la pièce d'appui de prothèse (10).

4. Pièce d'appui de prothèse selon une quelconque des revendications précédentes, une ou plusieurs des ouvertures (20) se prolongeant partiellement à travers l'épaisseur de la pièce d'appui de prothèse.

5. Pièce d'appui de prothèse selon la revendication 4, une ou plusieurs des ouvertures (20) étant formées par un évidement annulaire (110, 120).

6. Pièce d'appui de prothèse selon la revendication 5, une partie d'engagement (100, 130) pour l'engagement avec un dispositif d'engagement étant prévue à l'intérieur de l'évidement annulaire (110, 120).

7. Pièce d'appui de prothèse selon la revendication 4, une ou plusieurs des ouvertures (20) étant formée par une rainure annulaire (80, 90).

8. Pièce d'appui de prothèse selon la revendication 7, une partie d'engagement (100) pour l'engagement avec un dispositif d'engagement étant prévue à l'intérieur de l'espace annulaire défini par la rainure annulaire (80, 90).

9. Pièce d'appui de prothèse selon la revendication 6 ou 8, la partie d'engagement (100, 130) étant définie par un évidement ou par une saillie.

10. Pièce d'appui de prothèse selon la revendication 1, une ou plusieurs des ouvertures (20) partant d'une surface extérieure (50) ou d'une surface intérieure (40) de la pièce d'appui de prothèse, et la ou plusieurs ouvertures se prolongeant à travers l'épaisseur de la pièce d'appui de prothèse lors de la rupture sélective de l'élément frangible (70).

11. Pièce d'appui de prothèse selon une quelconque des revendications précédentes, le périmètre de la ou des ouvertures (20) sur la surface d'appui étant continu de sorte que les ouvertures soient entourées par la surface d'appui.

12. Pièce d'appui de prothèse selon une quelconque des revendications précédentes, le dispositif de fixation (30) comprenant une tête, et la tête du dispositif de fixation n'étant pas au ras de la surface d'appui, de sorte qu'un évidemment se forme dans la surface d'appui.

13. Pièce d'appui de prothèse selon une quelconque des revendications précédentes, un ou plusieurs bouchons étant placés dans la ou plusieurs ouvertures (20) dans la surface d'appui.

14. Pièce d'appui de prothèse selon une quelconque des revendications précédentes, la pièce d'appui de prothèse comprenant une couche unique de polyaryléthercétone (PAEK) renforcé à la fibre de carbone.
